# EUROPEAN PATENT SPECIFICATION

(11) **EP 4 368 136 B1**
(45) Date of publication and mention of the grant of the patent: **14.01.2026**
(21) Application number: 22846259.4
(22) Date of filing: 21.07.2022
(51) Int. Cl.: A61B 34/10, A61B 34/20, A61B 34/00, A61B 90/00

(54) **APPARATUS FOR DETERMINING INSERTION POSITION OF TROCAR ON THREE-DIMENSIONAL VIRTUAL PNEUMOPERITONEUM MODEL OF PATIENT**
VORRICHTUNG ZUR BESTIMMUNG DER EINSATZPOSITION EINES TROKARS AUF EINEM DREIDIMENSIONALEN VIRTUELLEN PNEUMOPERITONEUMMODELL EINES PATIENTEN
APPAREIL POUR DÉTERMINER LA POSITION D'INSERTION D'UN TROCART SUR UN MODÈLE VIRTUEL TRIDIMENSIONNEL DE PNEUMOPÉRITOINE D'UN PATIENT

(30) Priority: 21.07.2021 KR 20210095902
(43) Date of publication of application: 15.05.2024
(73) Proprietor: HUTOM CO., LTD., Seoul 04151 (KR)
(72) Inventor: HAN, Yejin, Seoul 06624 (KR); YOO, Hyoung Eun, Seoul 04137 (KR); KANG, Taewon, Incheon 22203 (KR); SUNG, Nak Jun, Seoul 04137 (KR)
(74) Representative: dompatent
(86) International application number: PCT/KR2022/010698
(87) International publication number: WO 2023/003389

(56) References cited:
- JP-A- 2011 113 056
- JP-A- 2011 113 056
- JP-A- 2014 132 980
- JP-A- 2020 096 783
- KR-A- 20140 112 207
- KR-B1- 102 013 837
- US-A1- 2020 113 637
- US-A1- 2020 297 422

## Description

### [TECHNICAL FIELD]

The present disclosure relates to an apparatus and method for determining an insertion position of a trocar on a three-dimensional virtual pneumoperitoneum model of a patient.

### [BACKGROUND ART]

Recently, when a surgery is performed in a hospital, instead of performing the surgery right away, the conditions of the patient before the surgery may be created in a 3D simulation (stereoscopic images), and then the surgery may be performed virtually under the same conditions as an actual surgery.

In such a virtual simulation surgery, a precise diagnosis may be established in advance. Accordingly, rather than relying on an intuition of a specialist, a plan may be established through a virtual simulation surgery and then even the smallest errors may be reduced.

However, this virtual simulation surgery has a problem of lacking realism. Additionally, in surgical operations, medical staff cannot perform virtual simulation surgeries under the same conditions as the actual surgeries.

In particular, when a minimally invasive surgery (for example, a robotic surgery or a laparoscopic surgery) is performed, an image identified by the medical staff in the simulation process and an image seen during the actual surgery are different and the uses of the tools are different when a photographing direction of a camera and an entry point of a surgical tool are different during the actual surgery and the virtual simulated surgery whereby the training may not have the same effect as the actual surgery.

Accordingly, an intra-abdominal structure may differ depending on the position of the camera to check the intra-abdominal structure, and thus, it is necessary to set a position of a trocar in the same way as during the actual surgery so that the camera and surgical tools may enter an interior of a virtual pneumoperitoneum model, to which pneumoperitoneum state (a state, in which gas is injected into the body of the patient to inflate the abdomen of the patient to facilitate surgery) is applied, during the virtual simulation surgery in the same way as during the actual surgery.

Publication US2020/113637 discloses a robotically-assisted surgical device capable of recognizing piercing accuracy required for piercing a port. Other technological background for the present invention is disclosed in the following publications:
US 2020/297422 A1
JP 2011 113056 A

### [DETAILED DESCRIPTION OF THE INVENTION]

### [TECHNICAL PROBLEM]

An aspect of the present disclosure is to determine a disposition position of a trocar in a pneumoperitoneum model of a patient, which is used in the same surgical simulation as during an actual surgery.

An aspect of the present disclosure also provides a surgical simulation environment, in which trocars are disposed in the same way as an actual surgery based on a virtual pneumoperitoneum model, in which an actual pneumoperitoneum condition of the patient is predicted whereby a surgical simulation may serve as an excellent rehearsal for the actual surgery.

The technical problems sought to be achieved in the present disclosure are not limited to the technical problems mentioned above, and other technical problems not mentioned may be clearly understood by those skilled in the art, to which the present disclosure pertains, from the description below.

### [TECHNICAL SOLUTION]

The presently claimed invention provides a computer program executing a method of determining an insertion position of a trocar according to claim 1, and an apparatus for determining an insertion position of a trocar according to claim 2. Further developments of the herein claimed invention are described in the dependent claims.

According to an aspect of the present disclosure, a method of determining an insertion position of a trocar on a three-dimensional virtual pneumoperitoneum model of a patient, the method being performed by a device includes displaying a user interface (UI) for setting the insertion position of the trocar on the virtual pneumoperitoneum model used in a surgery simulation, displaying the virtual pneumoperitoneum model on the UI, determining a position spaced apart from a navel of the virtual pneumoperitoneum model by a preset distance as an insertion position of a reference trocar, into which a camera (endoscope) is to be inserted, through the UI, determining an insertion position of at least one trocar on a surface of the virtual pneumoperitoneum model based on the determined insertion position of the reference trocar, through the UI, calculating and storing information on a distance between the insertion position of the reference trocar and the determined insertion position of the at least one trocar, on the surface of the virtual pneumoperitoneum model, and providing the stored distance information, on the UI, in real time during an actual surgery on the patient, and the distance information includes transverse and longitudinal distances between the insertion position of the reference trocar and the insertion position of the at least one trocar, and a diagonal distance for the transverse and longitudinal distances.

Furthermore, the UI includes a main screen area, in which the virtual pneumoperitoneum model is displyed on a plan view, and a preview screen area, in which an internal image of the virtual pneumoperitoneum model captured through the camera inserted through the reference trocar is displayed.

Furthermore, the insertion position of the at least one trocar may be adjusted on the main screen area through the UI.

Furthermore, in the preview screen area, an image of a tool inserted into the at least one trocar entering an interior of the virtual pneumoperitoneum model may be captured through the camera and displayed in real time.

Furthermore, in the main screen area, the stored distance information and information on a tool inserted through the at least one trocar may be displayed

Furthermore, in the preview screen area, port numbers of the reference trocar and the at least one trocar may be displayed, and whether a corresponding tool collides with an internal organ when the corresponding tool enters the interior of the virtual pneumoperitoneum model at positions of the reference trocar and the at least one trocar may be displayed.

Furthermore, the determining of the insertion position of the at least one trocar includes calculating values of differences from a first point corresponding to the insertion position of the reference trocar to a second point corresponding to the insertion position of the at least one trocar in transverse, longitudinal, and diagonal directions, radiating a preset unit of rays in the transverse, longitudinal, and diagonal directions, storing three-dimensional positions, which the rays reach, on the surface of the virtual pneumoperitoneum model, and determining the insertion position of the at least one trocar by summing the distances of the stored three-dimensional positions.

Furthermore, the determining of the insertion position of the at least one trocar may include changing the insertion position of the at least one trocar based on a changed position of the second point when the position of the second point is changed.

According to another aspect of the present disclosure, an apparatus of determining an insertion position of a trocar on a three-dimensional virtual pneumoperitoneum model of a patient includes an acquisition part that acquires the virtual pneumoperitoneum model used in a surgery simulation, a display part displays a user interface (UI) for setting an insertion position of the trocar on the virtual pneumoperitoneum model, and a processor that displays the virtual pneumoperitoneum model on the UI, determines a position spaced apart from a navel of the virtual pneumoperitoneum model by a preset distance as an insertion position of a reference trocar, into which a camera (endoscope) is to be inserted, through the UI, determines an insertion position of at least one trocar on a surface of the virtual pneumoperitoneum model based on the determined insertion position of the reference trocar, through the UI, calculates and store information on a distance between the insertion position of the reference trocar and the determined insertion position of the at least one trocar, on the surface of the virtual pneumoperitoneum model, and provides the stored distance information, on the UI, in real time during an actual surgery on the patient, and the distance information includes transverse and longitudinal distances between the insertion position of the reference trocar and the insertion position of the at least one trocar, and a diagonal distance for the transverse and longitudinal distances.

Other specific details of the invention are included in the detailed description and the drawings.

### [ADVANTAGEOUS EFFECTS OF THE INVENTION]

According to the present disclosure as above, the present disclosure may determine a disposition position of the trocar on the pneumoperitoneum model of the patient, which is used in the same surgical simulation as during the actual surgery, the body of the patient, the positions of the surgical tools, the distances to organs, the angles, and the like may be predicted with a high accuracy through the same camera (endoscope) as the actual surgery.

The effects of the present disclosure are not limited to the effects mentioned above, and other effects not mentioned may be clearly understood by those skilled in the art, to which the present disclosure pertains, from the description below.

### [DESCRIPTION OF THE DRAWINGS]

FIG. 1 is a view illustrating an apparatus for determining an insertion position of a trocar on a three-dimensional virtual pneumoperitoneum model of a patient according to the present disclosure;
FIG. 2 is an exemplary view illustrating a UI for setting an insertion position of a trocar on a virtual pneumoperitoneum model used in a surgery simulation according to the present disclosure;
FIGS. 3A and 3B are exemplary views illustrating determining an insertion position of a reference trocar according to the present disclosure;
FIG. 4 is an exemplary view illustrating determining an insertion position of at least one trocar according to the present disclosure;
FIGS. 5A and 5B are exemplary views illustrating determining insertion positions of a first trocar and a second trocar according to the present disclosure;
FIG. 6 is an exemplary view illustrating determining insertion positions of a reference trocar and first to fourth trocars according to the present disclosure; and
FIG. 7 is a flowchart illustrating a process of determining an insertion position of a trocar on a three-dimensional virtual pneumoperitoneum model of a patient according to the present disclosure.

### [BEST MODE]

The advantages and features of the present disclosure, and how to achieve them, will become clear by referring to the embodiments described in detail below along with the accompanying drawings. However, the present disclosure is not limited to the embodiments disclosed below and may be implemented in various different forms, and the embodiments only ensure that the disclosure of the present disclosure is complete and fully inform those skilled in the art, to which the present disclosure pertains of the scope of the present disclosure, and the present disclosure is only defined by the scope of the claims.

The terminology used herein is intended to describe embodiments and is not intended to limit the present disclosure. As used herein, singular forms also include plural forms, unless specifically stated otherwise in the context. As used in the specification, "comprises" and/or "comprising" does not exclude the presence or addition of one or more other elements in addition to the mentioned elements. The same reference numerals refer to the same elements throughout the specification, and "and/or" includes each and every combination of one or more of the referenced elements. Although "first", "second", and the like are used to describe various components, these components are of course not limited by these terms. These terms are merely used to distinguish one component from another. Therefore, of course, the first component mentioned below may also be the second component within the technical idea of the present disclosure.

Unless otherwise defined, all terms (including technical and scientific terms) used in this specification may be used with a meaning commonly understood by those skilled, in the art to which the present disclosure pertains. Furthermore, terms defined in commonly used dictionaries are not interpreted ideally or excessively unless clearly specifically defined.

Hereinafter, embodiments of the present disclosure will be described in detail with reference to the attached drawings.

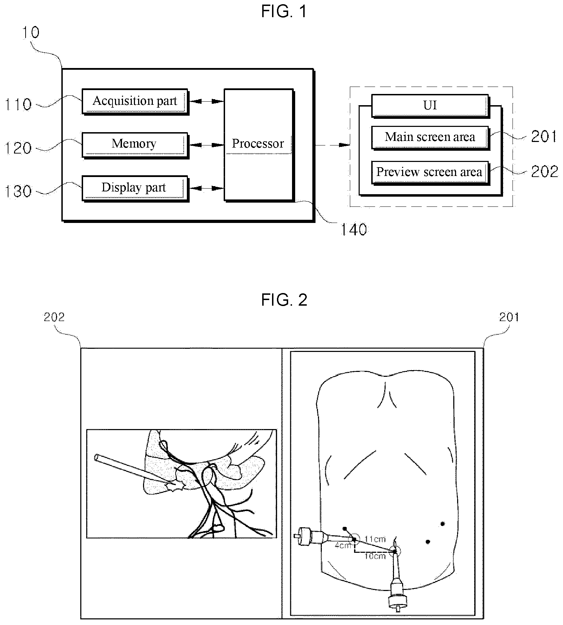

FIG. 1 is a view illustrating an apparatus 10 for determining an insertion position of a trocar on a three-dimensional virtual pneumoperitoneum model of a patient according to the present disclosure.

FIG. 2 is an exemplary view illustrating a UI for setting the insertion position of the trocar on the virtual pneumoperitoneum model used in a surgery simulation according to the present disclosure.

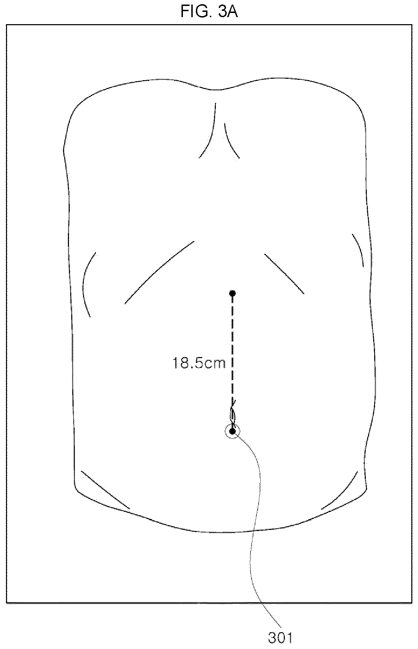

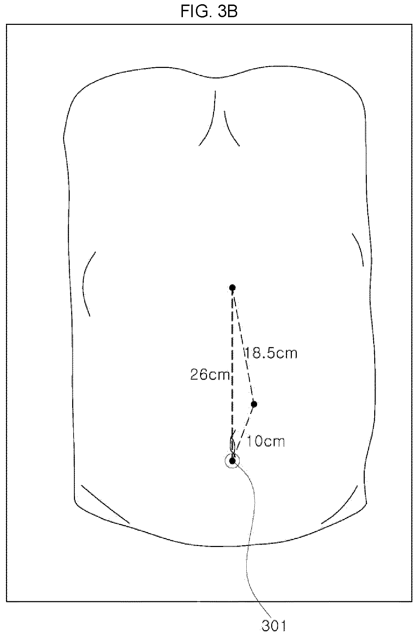

FIGS. 3A and 3B are exemplary views illustrating determining an insertion position of a reference trocar according to the present disclosure.

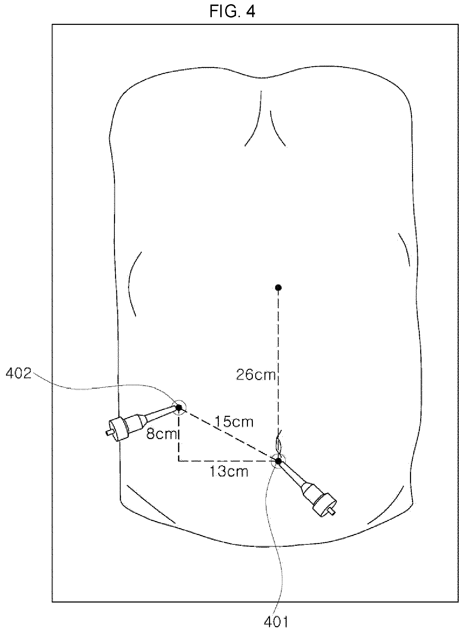

FIG. 4 is an exemplary view illustrating determining an insertion position of at least one trocar according to the present disclosure.

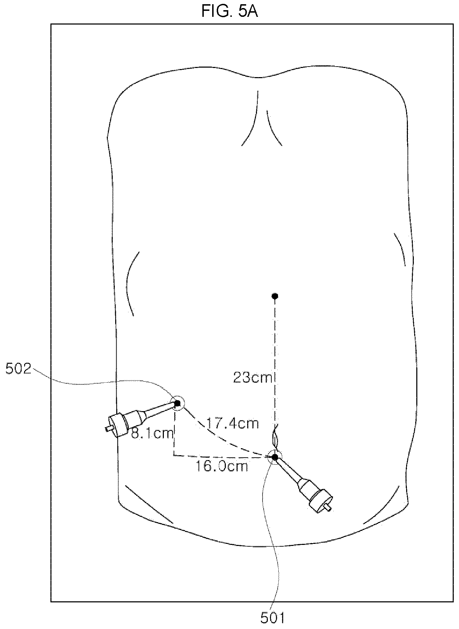

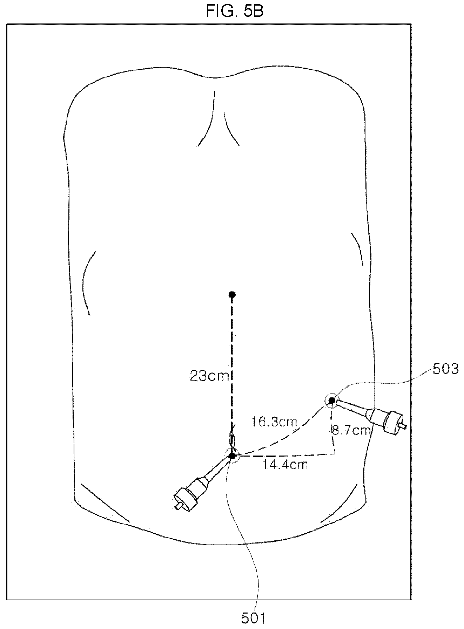

FIGS. 5A and 5B are exemplary views illustrating determining insertion positions of a first trocar and a second trocar according to the present disclosure.

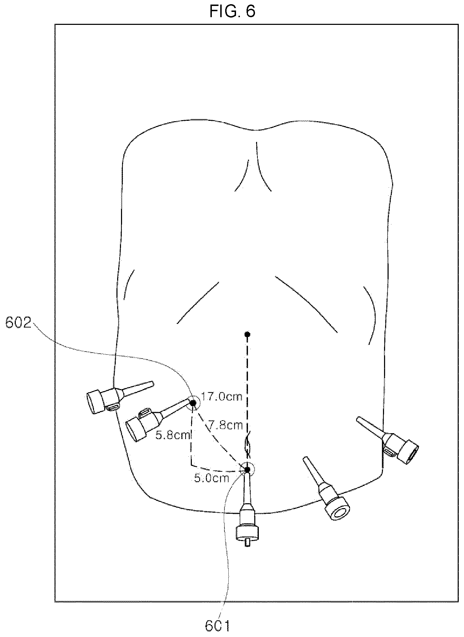

FIG. 6 is an exemplary view illustrating determining the insertion positions of the reference trocar and first to fourth trocars according to the present disclosure.

Hereinafter, the apparatus 10 for determining the insertion position of the trocar on the three-dimensional virtual pneumoperitoneum model of a patient according to the present disclosure will be described with reference to FIGS. 1 to 6.

In detail, when a minimally invasive surgery (e.g., laparoscopic surgery or a robotic surgery) is performed, a camera that entered an interior of a body through a trocar that passes through the body may perform a surgery with a surgical tool that entered through one or more trocars inserted into another position while identifying some ranges of the interior of the body.

Then, to ensure a space for surgical tools to move in the interior of the body, a preset gas (e.g., carbon dioxide) may be injected into the body (e.g., the space between the abdominal walls when an abdominal surgery is performed) during the minimally invasive surgery.

Medical staff wants to prepare for various variables that may occur during an actual surgery by simulating a surgery in advance before the actual surgery, and as a countermeasure, a virtual surgery simulation may be provided in the same virtual surgery environment as the actual surgery.

Because the minimally invasive surgery is performed while being identified only with the camera (i.e., endoscope) that is inserted into the interior of the body, no practice effect may be obtained at all because the images provided to the medical staff during the actual surgery are completely different when the medical staff performs the actual surgery after they practice with images displayed in a completely different position or direction in a virtual simulation.

In particular, even when a virtual body model that is undulated in the same manner as a body condition of the patient during the physical surgery is modeled, no practice effect may be obtained by performing a practice with tools positioned at a different position while a completely different image is seen when the camera enters in a different position or direction or the surgical tool enters in a different position or direction.

Accordingly, the apparatus 10 needs to set the position of the trocar so that the camera and surgical tools enter the virtual pneumoperitoneum model in the same manner as during the actual surgery.

The apparatus 10 may determine the disposition position of the trocar on the pneumoperitoneum model of the patient, which is used in the same surgery simulation as during the actual surgery whereby a view of the interior of the body and the positions of the surgical tools, and a distance and an angle to an organ may be predicted with a high precision through the same camera (endoscope) as during the actual surgery of the patient.

Here, the apparatus 10 may include various devices that may perform computational processing and provide results to the user.

That is, the apparatus 10 may be in the form of a computer. In more detail, the computer may include various devices that may perform computational processing and provide results to the user.

For example, computers may include not only desktop PCs and laptops (notebooks), but also smart phones, tablet PCs, cellular phones, personal communication service (PCS) phones, and synchronous/asynchronous international mobile telecommunication-2000 (IMT-2000) mobile terminals, palm personal computers (palm PCs), and personal digital assistants (PDAs). Furthermore, when a head mounted display (HMD) device includes computing functions, the HMD device may be a computer.

Furthermore, a computer may be a server that receives requests from clients and performs information processing.

Furthermore, the apparatus 10 may include an acquisition part 110, a memory 120, a display part 130, and a processor 140. Here, the apparatus 10 may include fewer or more components than those illustrated in FIG. 1.

The acquisition part 110 may include one or more modules that enable wireless communication between the apparatus 10 and an external device (not illustrated), between the apparatus 10 and an external server (not illustrated), or between the apparatus 10 and a communication network (not illustrated).

Here, the acquisition part 110 may include one or more modules that connect the apparatus 10 to one or more networks.

The acquisition part 110 may acquire the virtual pneumoperitoneum model used in a surgery simulation from the external server (not illustrated) or the memory 120.

Here, the external apparatus (not illustrated) may be medical imaging equipment that captures medical image data (hereinafter referred to as abdominal 3D image data). Here, the medical image data may include all medical images that may be implemented as a three-dimensional model of the body of the patient.

Furthermore, the medical image data may include at least one of computed tomography (CT) images, magnetic resonance imaging (MRI), and positron emission tomography (PET) images.

Furthermore, the external server (not illustrated) may be a server that stores a virtual pneumoperitoneum model for each patient, medical data for each patient, and the like for a plurality of patients. Here, the medical data for each patient may include data on at least one of the age, the gender, the height, the weight, the body mass index, and the childbirth status of the patient.

Furthermore, a communication network (not illustrated) may transmit and receive various information between the apparatus 10, an external device (not illustrated), and an external server (not illustrated). Various types of communication networks may be used, and for example, wireless communication methods, such as wireless LAN (WLAN), Wi-Fi, Wibro, Wimax, and high speed downlink packet access (HSDPA), or wired communication methods, such as Ethernet, xDSL (ADSL, VDSL), hybrid fiber coax (HFC), fiber to the curb (FTTC), and fiber to the home (FTTH), may be used.

Meanwhile, the communication network is not limited to the communication methods presented above, and may include all other types of communication methods that are widely known or will be developed in the future in addition to the communication methods described above.

The memory 120 may store data that support various functions of the apparatus 10. The memory 120 may store a number of application programs (or applications) that run on the apparatus 10, data for operating the apparatus 10, and commands. At least some of these application programs may exist for basic functions of the apparatus 10. Meanwhile, the application programs may be stored in the memory 120, installed on the apparatus 10, and driven by the processor 140 to perform the operation (or function) of the apparatus 10.

Furthermore, the memory 120 may include a plurality of processes for determining the insertion position of the trocar on the three-dimensional virtual pneumoperitoneum model of a patient according to the present disclosure. Here, the plurality of processes will be described later when an operation of the processor 140 is described.

The memory 120 may store a virtual pneumoperitoneum model for each patient. Here, the virtual pneumoperitoneum model may be created and stored through the processor 140, or may be acquired and stored from the external server (not illustrated).

The display part 130 may implement a touchscreen by forming a mutual layer structure or being integrated with a touch sensor. The touchscreen may provide an input interface between the apparatus 10 and the user.

The display part 130 may display a user interface for setting the insertion position of the trocar on the virtual pneumoperitoneum model.

In addition to operations related to the application program, the processor 140 may generally control an overall operation of the apparatus 10. The processor 140 may provide appropriate information or functions to the user or process it by processing signals, data, information, and the like that are input or output through the components discussed above, or by running an application program stored in the memory 120.

Furthermore, the processor 140 may control at least some of the components discussed together with FIG. 1 to run an application program stored in the memory 120. Furthermore, the processor 140 may operate in combination with at least two or more of the components included in the apparatus 10 to run the application program.

The processor 140 may display a user interface (UI) for setting the insertion position of the trocar on the virtual pneumoperitoneum model used in a surgery simulation.

Here, the processor 140 may display a UI for setting the insertion position of the trocar on the virtual pneumoperitoneum model based on, among the plurality of processes, a first process through the display part 130.

Furthermore, the processor 140 may display the virtual pneumoperitoneum model on the UI. Here, the processor 140 may display the virtual pneumoperitoneum model on the UI based on, among the plurality of processes, a second process.

Referring to FIG. 2, the UI may include a main screen area 201 and a preview screen area 202.

In detail, the main screen area 201 may be an area, in which the virtual pneumoperitoneum model is displayed in a plan view. That is, the main screen area 201 may be a screen that views a surface of the pneumoperitoneum model from a top.

Here, the insertion position of the at least one trocar on the surface of the virtual pneumoperitoneum model on the main screen area 201 may be adjustable through the UI.

Furthermore, the main screen area 201 may display stored information on a distance between the insertion position of the reference trocar and the insertion position of at least one trocar, for which the insertion position is determined, and information on a tool that is inserted through the at least one trocar.

Here, the distance information may include transverse and longitudinal distances (10 cm and 4 cm) between the insertion position of the reference trocar and the insertion position of the at least one trocar, and a diagonal distance (11 cm) for the transverse and longitudinal distances.

The preview screen area 202 may be an area, in which an internal image of the virtual pneumoperitoneum model, which is captured through the camera inserted through the reference trocar is displayed.

Furthermore, the preview screen area 202 may display in real time the image of the tool inserted into the at least one trocar entering the interior of the virtual pneumoperitoneum model, which is captured by the camera.

Furthermore, the preview screen area 202 may display the port numbers of the reference trocar and the at least one trocar.

Here, the port numbers may be displayed to provide the same interface as the robotic surgery console. That is, in the robotic surgery, a number is assigned to the trocar that is connected to a robot arm, and this may be indicated as a port number. Then, the port number may be numbered as 1 from the leftmost position in the field of view of an operator.

Furthermore, the preview screen area 202 may display whether the tool collides with an internal organ when the tool enters the interior of the virtual pneumoperitoneum model at the positions of the reference trocar and the at least one trocar.

Here, the processor 140 may simultaneously output, on the UI, the main screen area 201, in which the surface of the pneumoperitoneum model is viewed from a top, and the preview screen area 202, in which the surface of the pneumoperitoneum model is viewed through the inserted camera (endoscope).

Accordingly, the processor 140 may be configured to provide an appropriate position while synchronizing and outputting the position on the abdominal surface of the trocar in the main screen area 201 and the position in the preview screen area 202, which is visible through the inserted camera (endoscope).

Furthermore, the processor 140 may display information on whether a collision occurs when the tool is inserted at the insertion position of the trocar through blinking. Here, the information on whether a collision occurs may be whether the surgical tool at the insertion position of the trocar reaches the organ.

Accordingly, the processor 140 may identify whether the organ may be reached when the trocar is inserted and the tool (surgical tool) is inserted at the insertion position of the trocar and may provide the information to the user.

Through the UI, the processor 140 may determine a position that is spaced apart from the navel of the virtual pneumoperitoneum model by a preset distance as the insertion position of the reference trocar, at which the camera (endoscope) is to be inserted.

Here, the processor 140 may determine the insertion position of the reference trocar, at which the camera (endoscope) is to be inserted, through the UI based on, among the plurality of processes, a third process.

Referring to FIG. 3A to 3B, the processor 140 may determine the insertion position of a reference trocar 301, at which the camera is to be inserted, to be around 1 cm below the navel through the UI.

Furthermore, the processor 140 may insert the camera through the reference trocar 301 while visually identifying the positions of and the distance between the solar plexus and the navel.

Here, referring to FIG. 3A, it may be seen that a surface distance between the position of the solar plexus and the position of the navel is 18.5 cm, and referring to FIG. 3B, it may be seen that after the camera is inserted into the reference trocar 301, a surface distance between the inserted camera and the insertion position below the navel, at which the camera is inserted, is 10 cm, a surface distance between the camera and the solar plexus is 18.5 cm, and a surface distance between the solar plexus and the insertion position, at which the camera is inserted, below the navel is 26 cm.

The processor 140 may determine the insertion position of the at least one trocar on the surface of the virtual pneumoperitoneum model based on the determined insertion position of the reference trocar through the UI.

Here, the processor 140 may determine the insertion position of the at least one trocar through the UI based on, among the plurality of processes, a fourth process.

Referring to FIG. 4, in detail, the processor 140 may select a second point 402 corresponding to the insertion position of the at least one trocar from a first point 401 corresponding to the insertion position of the reference trocar.

Next, the processor 140 may calculate values of differences from the first point 401 to the second point 402 in the horizontal, vertical, and diagonal directions. Here, the values of the differences in the horizontal, vertical, and diagonal directions may be distances in the horizontal, vertical, and diagonal directions on the surface of the three-dimensional virtual pneumoperitoneum model.

Next, the processor 140 may emit a preset unit of rays in the transverse direction (the "X" direction), the longitudinal direction (the "Y" direction), and the diagonal directions.

Here, the processor 140 may generate a list of collision points of rays in the transverse direction (the "X" direction), the longitudinal direction (the "Y" direction), and the diagonal directions.

Next, the processor 140 may store a three-dimensional position, at which the ray reaches the surface of the virtual pneumoperitoneum model. In detail, the processor 140 may determine and store the 3D position, at which the ray arrives, based on the list of the collision points.

Next, the processor 140 may determine the insertion position of the at least one trocar by summing the distances of the stored 3D positions.

Here, when the position of the second point is changed, the processor 140 may also change the insertion position of the at least one trocar based on the changed position of the second point.

As an example, referring to FIG. 5A, the processor 140 may calculate the values of the differences from a first point 501 corresponding to the insertion position of the reference trocar to a second point 502 corresponding to the insertion position of the first trocar located on the left side of the first point 510 in the transverse, longitudinal, and diagonal directions, and may emit a preset unit of rays in the respective directions.

Here, the values of the differences in the transverse, longitudinal, and diagonal directions (the transverse direction (16.0 cm), the longitudinal direction (8.1 cm), and the diagonal directions (17.4 cm)) may be displayed in real time on the UI.

The differences of the values in the transverse, longitudinal, and diagonal directions displayed in real time on the UI may have the same effect as when a doctor checks the position by pressing the stomach of the patient during an actual surgery.

Furthermore, the processor 140 may store the 3D position, at which the ray reaches the surface of the virtual pneumoperitoneum model, and may determine the insertion position of the first trocar by summing the distances of the stored 3D positions.

As another example, referring to FIG. 5B, the processor 140 may calculate the values (the transverse direction (14.4 cm) and the longitudinal direction (87. cm) from the first point 501 corresponding to the insertion position of the reference trocar to a third point 503 corresponding to the insertion position of the second trocar located on the right side of the first point 501, and the diagonal lines (16.3 cm), and may emit a preset unit of rays in the respective directions.

Here, the values of the differences in the transverse, longitudinal, and diagonal directions may be displayed in real time on the UI.

Furthermore, the processor 140 may store the 3D position, at which the ray reaches the surface of the virtual pneumoperitoneum model, and may determine the insertion position of the second trocar by summing the distances of the stored 3D positions.

As another example, referring to FIG. 6, the processor 140 may determine the insertion positions of the first and second trocars on the left side, and the third and fourth trocars on the right side, based on the reference trocar.

Here, the first trocar, the second trocar, and the fourth trocar may be robotic trocars, and the third trocar may be a laparoscopic trocar. As such, the robotic trocar may be a trocar for a robotic surgery, and the laparoscopic trocar may be a trocar for a laparoscopic surgery.

In detail, the processor 140 may calculate the values of the differences from a first point 601 corresponding to the insertion position of the reference trocar to a second point 602 corresponding to the insertion point of, among the first to fourth trocars, the first trocar located on the left side of the first point 601 in the transverse, longitudinal, and diagram directions, and may emit a preset unit of rays in the respective directions.

Furthermore, the processor 140 may store the 3D position, at which the ray reaches the surface of the virtual pneumoperitoneum model, and may determine the insertion position of the first trocar by summing the distances of the stored 3D positions. Here, the first trocar may be a robotic trocar.

The processor 140 may calculate and store information on the distance between the insertion position of the reference trocar and the determined insertion position of at least one trocar on the surface of the virtual pneumoperitoneum model.

Here, the processor 140 may calculate and store the information on the distance between the reference trocar and the at least one trocar based on, among a plurality of processes, a fifth process.

The distance information may include transverse and longitudinal distances between the insertion position of the reference trocar and the insertion position of the at least one trocar, and diagonal distances for the transverse and longitudinal distances.

The processor 140 may provide the stored distance information on the UI in real time during the actual surgery on the patient.

Here, the processor 140 may provide the stored distance information on the UI in real time based on, among a plurality of processes, a sixth process.

Accordingly, during the actual surgery, instead of positioning the trocar by pressing the abdomen of the patient, the expert may insert the trocar at the corresponding position based on the preset insertion position on the UI.

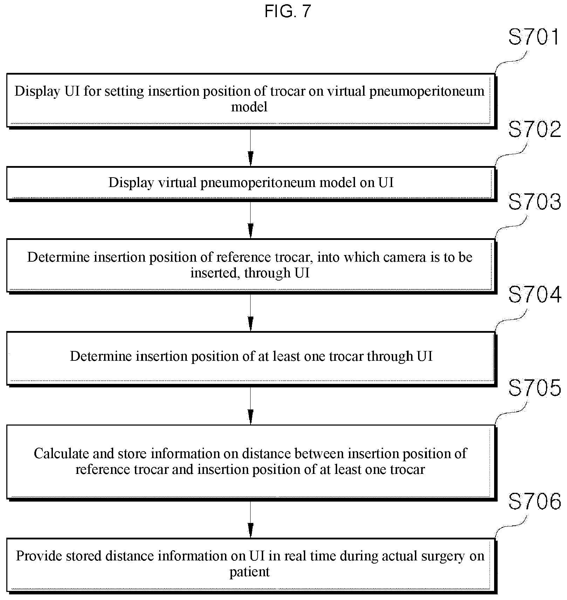

FIG. 7 is a flowchart illustrating a process of determining an insertion position of a trocar on a three-dimensional virtual pneumoperitoneum model of a patient according to the present disclosure. Here, an operation of the processor 140 may be performed in the apparatus 10.

The processor 140 may display the user interface (UI) for setting the insertion position of the trocar on the virtual pneumoperitoneum model (S701).

The processor 140 may display the virtual pneumoperitoneum model on the UI (S702).

In more detail, the processor 140 may display the UI for setting the insertion position of the trocar on the virtual pneumoperitoneum model used for surgery simulation for each patient.

Here, the UI may include the main screen area, in which the virtual pneumoperitoneum model is displayed in a plan view, and a preview screen area, in which an internal image of the virtual pneumoperitoneum model, which is captured through the camera inserted through the reference trocar, is displayed.

In detail, in the preview screen area, the image of the tool inserted into the at least one trocar that enters the interior of the virtual pneumoperitoneum model may be captured through the camera and displayed in real time.

Furthermore, the preview screen area may display the port numbers of the reference trocar and the at least one trocar, and display whether there is a collision with an internal organ when the corresponding tool enters the interior of the virtual pneumoperitoneum model at the positions of the reference trocar and the at least one trocar.

Furthermore, the main screen area may display the stored distance information and information on the tool inserted through the at least one trocar.

Here, the distance information may include the transverse and longitudinal distances between the insertion position of the reference trocar and the insertion position of the at least one trocar, and the diagonal distance for the transverse and longitudinal distances.

Furthermore, the insertion position of the at least one trocar may be adjustable on the main screen area through the UI.

The processor 140 may determine the insertion position of the reference trocar, into which the camera (endoscope) is to be inserted, through the UI (S703).

In more detail, the processor 140 may determine, through the UI, a position that is spaced apart from the navel of the virtual pneumoperitoneum model by a preset distance as the insertion position of the reference trocar, at which the camera is to be inserted.

The processor 140 may determine the insertion position of at least one trocar through the UI (S704).

In more detail, the processor 140 may determine the insertion position of at least one trocar on the surface of the virtual pneumoperitoneum model based on the determined insertion position of the reference trocar, through the UI.

In detail, the processor 140 may calculate the values of the differences from the first point corresponding to the insertion position of the reference trocar to the second point corresponding to the insertion position of the at least one trocar in the transverse, longitudinal, and diagonal directions.

Furthermore, the processor 140 may radiate a preset unit of rays in the transverse, longitudinal, and diagonal directions, and may store the three-dimensional position, at which the rays reach the surface of the virtual pneumoperitoneum model.

Accordingly, the processor 140 may determine the insertion position of the at least one trocar by summing the distances of the stored 3D positions.

Here, when the position of the second point is changed, the processor 140 may also change the insertion position of the at least one trocar based on the changed position of the second point.

The processor 140 may calculate and store the information on the distance between the insertion position of the reference trocar and the determined insertion position of at least one trocar (S705).

In more detail, the processor 140 may calculate and store the information on the distance between the insertion position of the reference trocar and the determined insertion position of at least one trocar on the surface of the virtual pneumoperitoneum model.

The processor 140 may provide the stored distance information on the UI in real time during the actual surgery on the patient (S706).

FIG. 7 illustrates that operations S701 to S706 are sequentially executed, but this is merely an illustrative description of the technical idea of this embodiment, and those skilled in the art will understand that the sequence described in FIG. 7 may be changed to be executed or one or more of operations S701 to 706 may be executed in parallel through various corrections and modifications without departing from the essential characteristics of the embodiment, and thus, FIG. 7 is not limited to a time-serial sequence.

The method according to an embodiment of the present disclosure described above may be implemented as a program (or application) and stored in a medium to be executed in combination with a computer, which is hardware. Here, the computer may be the apparatus 10 described above.

The above-mentioned program may include codes coded in a computer language, such as C, C++, JAVA, and a machine language, which may be read by a processor (CPU) of the computer through a device interface of the computer for the computer to read the program and execute the methods implemented in the program. The codes may include functional codes related to functions that define the necessary functions for executing the methods, and include control codes related to execution procedures that are necessary for the processor of the computer to execute the functions according to specific procedures. In addition, the codes may further include memory reference-related codes that indicate at which position (address) in the an internal or external memory of the computer additional information or media required for the processor of the computer to execute the above functions have to be referenced. In addition, when the processor of the computer needs to communicate with any other remote computer or server to execute the above functions, the code may further include communication-related codes regarding how to communicate with any other remote computer or server by using the communication module or communication should be performed and what information or media have to be transmitted and received during communication.

The operations of the method or algorithm described in connection with the embodiments of the present disclosure may be implemented directly in hardware, implemented as a software module executed by hardware, or a combination thereof. The software module may reside on a random access memory (RAM), a read only memory (ROM), an erasable programmable ROM (EPROM), an electrically erasable programmable ROM (EEPROM), a flash memory, a hard disk, an attachable disk, a CD-ROM, or a computer-readable recording medium in a form that is well known in the art, to which the present disclosure pertains.

Until now, the embodiments of the present disclosure have been described with reference to the attached drawings, but it may be understood that those skilled in the art in the field, to which the present disclosure pertains, will understand that the present disclosure may be implemented in another specific form without changing the technical idea or essential features. Therefore, the embodiments described above should be understood in all respects as illustrative and not restrictive.

## Claims

1. A computer program combined with a computer as hardware, and stored in a computer-readable recording medium to execute the method of determining an insertion position of a trocar on a three-dimensional virtual pneumoperitoneum model of a patient, the method being performed by a device, the method comprising:
displaying a user interface (UI) for setting the insertion position of the trocar on the virtual pneumoperitoneum model used in a surgery simulation;
displaying the virtual pneumoperitoneum model on the UI;
determining a position spaced apart from a navel of the virtual pneumoperitoneum model by a preset distance as an insertion position of a reference trocar, into which a camera (endoscope) is to be inserted, through the UI;
determining an insertion position of at least one trocar on a surface of the virtual pneumoperitoneum model based on the determined insertion position of the reference trocar, through the Ul;
calculating and storing information on a distance between the insertion position of the reference trocar and the determined insertion position of the at least one trocar, on the surface of the virtual pneumoperitoneum model; and
providing the stored distance information, on the UI, in real time during an actual surgery on the patient,
wherein the distance information includes transverse and longitudinal distances between the insertion position of the reference trocar and the insertion position of the at least one trocar, and a diagonal distance for the transverse and longitudinal distances.

2. An apparatus for determining an insertion position of a trocar on a three-dimensional virtual pneumoperitoneum model of a patient, the apparatus comprising:
an acquisition part (110) configured to acquire the virtual pneumoperitoneum model used in a surgery simulation;
a display part (130) configured to display a user interface (UI) for setting the insertion position of the trocar on the virtual pneumoperitoneum model; and
a processor (140) configured to:
display the virtual pneumoperitoneum model on the Ul;
determine a position spaced apart from a navel of the virtual pneumoperitoneum model by a preset distance as an insertion position of a reference trocar, into which a camera is to be inserted, through the UI;
determine an insertion position of at least one trocar on a surface of the virtual pneumoperitoneum model based on the determined insertion position of the reference trocar, through the UI;
calculate and store information on a distance between the insertion position of the reference trocar and the determined insertion position of the at least one trocar, on the surface of the virtual pneumoperitoneum model; and
provide the stored distance information, on the UI, in real time during an actual surgery on the patient,
wherein the distance information includes transverse and longitudinal distances between the insertion position of the reference trocar and the insertion position of the at least one trocar, and a diagonal distance for the transverse and longitudinal distances.

3. The apparatus of claim 2, wherein the UI includes a main screen area (201), in which the virtual pneumoperitoneum model is displayed on a plan view, and a preview screen area (202), in which an internal image of the virtual pneumoperitoneum model captured through the camera inserted through the reference trocar is displayed.

4. The apparatus of claim 3, wherein the insertion position of the at least one trocar is adjusted on the main screen area through the UI.

5. The apparatus of claim 3, wherein in the preview screen area, an image of a tool inserted into the at least one trocar entering an interior of the virtual pneumoperitoneum model is captured through the camera and displayed in real time.

6. The apparatus of claim 3, wherein in the main screen area, the stored distance information and information on a tool inserted through the at least one trocar are displayed, and
wherein in the preview screen area, port numbers of the reference trocar and the at least one trocar are displayed, and whether a corresponding tool collides with an internal organ when the corresponding tool enters the interior of the virtual pneumoperitoneum model at positions of the reference trocar and the at least one trocar.

7. The apparatus of claim 2, wherein the processor calculates values of differences from a first point corresponding to the insertion position of the reference trocar to a second point corresponding to the insertion position of the at least one trocar in transverse, longitudinal, and diagonal directions when the insertion position of the at least one trocar is determined, radiates a preset unit of rays in the transverse, longitudinal, and diagonal directions, stores three-dimensional positions, which the rays reach, on the surface of the virtual pneumoperitoneum model, and determines the insertion position of the at least one trocar by summing the distances of the stored three-dimensional positions.

8. The apparatus of claim 7, wherein the processor changes the insertion position of the at least one trocar based on a changed position of the second point when the position of the second point is changed, when the insertion position of the at least one trocar is determined.

## Patentansprüche

1. Computerprogramm, kombiniert mit einem Computer als Hardware und gespeichert in einem computerlesbaren Aufzeichnungsmedium, um ein Verfahren zur Bestimmung einer Einsetzposition eines Trokars auf einem dreidimensionalen virtuellen Pneumoperitoneum-Modell eines Patienten durchzuführen, wobei das Verfahren durch eine Vorrichtung durchgeführt wird, wobei das Verfahren die folgenden Schritte aufweist:
Anzeigen einer Benutzerschnittstelle (UI) zum Einstellen der Einsetzposition des Trokars auf dem virtuellen Pneumoperitoneum-Modell, das in einer Operationssimulation verwendet wird;
Anzeigen des virtuellen Pneumoperitoneum-Modells auf der UI;
Bestimmen einer Position, die von einem Nabel des virtuellen Pneumoperitoneum-Modells um eine vorgegebene Entfernung beabstandet ist, als eine Einsetzposition eines Referenztrokars, in welchen eine Kamera (Endoskop) eingeführt werden soll, über die UI;
Bestimmen einer Einsetzposition mindestens eines Trokars auf einer Fläche des virtuellen Pneumoperitoneum-Modells basierend auf der bestimmten Einsetzposition des Referenztrokars über die UI;
Berechnen und Speichern von Informationen über eine Entfernung zwischen der Einsetzposition des Referenztrokars und der bestimmten Einsetzposition des mindestens einen Trokars auf der Oberfläche des virtuellen Pneumoperitoneum-Modells; und
Bereitstellen der gespeicherten Entfernungsinformationen auf der UI in Echtzeit während einer tatsächlichen Operation an einem Patienten,
wobei die Entfernungsinformationen Quer- und Längsentfernungen zwischen der Einsetzposition des Referenztrokars und der Einsetzposition des mindestens einen Trokars und eine diagonale Entfernung für die Quer- und Längsentfernungen aufweisen.

2. Vorrichtung zur Bestimmung einer Einsetzposition eines Trokars auf einem virtuellen dreidimensionalen Pneumoperitoneum-Modell eines Patienten, wobei die Vorrichtung aufweist:
einen Erfassungsteil (110), der dazu ausgebildet ist, das in einer Operationssimulation verwendete virtuelle Pneumoperitoneum-Modell zu erfassen;
einen Anzeigeteil (130), der zum Anzeigen einer Benutzerschnittstelle (UI) zum Einstellen der Einsetzposition des Trokars auf dem virtuellen Pneumoperitoneum-Modell ausgebildet ist; und
einen Prozessor (140), der dazu ausgebildet ist,
das virtuelle Pneumoperitoneum-Modells auf der UI anzuzeigen;
eine Position zu bestimmen, die von einem Nabel des virtuellen Pneumoperitoneum-Modells um eine vorgegebene Entfernung beabstandet ist, als eine Einsetzposition eines Referenztrokars, in welchen eine Kamera (Endoskop) eingeführt werden soll, über die UI;
eine Einsetzposition mindestens eines Trokars auf einer Fläche des virtuellen Pneumoperitoneum-Modells basierend auf der bestimmten Einsetzposition des Referenztrokars über die UI zu bestimmen;
Informationen über eine Entfernung zwischen der Einsetzposition des Referenztrokars und der bestimmten Einsetzposition des mindestens einen Trokars auf der Oberfläche des virtuellen Pneumoperitoneum-Modells zu berechnen und zu speichern; und
die gespeicherten Entfernungsinformationen auf der UI in Echtzeit während einer tatsächlichen Operation an einem Patienten bereitzustellen,
wobei die Entfernungsinformationen Quer- und Längsentfernungen zwischen der Einsetzposition des Referenztrokars und der Einsetzposition des mindestens einen Trokars und eine diagonale Entfernung für die Quer- und Längsentfernungen aufweisen.

3. Vorrichtung nach Anspruch 2, bei welcher die UI einen Hauptbildschirmbereich (201), in welchem das virtuelle Pneumoperitoneum-Modell in Draufsicht angezeigt wird, und einen Vorschaubildschirmbereich (202), in welchem ein inneres Bild des virtuellen Pneumoperitoneum-Modells angezeigt wird, welches durch die durch den Referenztrokar eingeführte Kamera aufgenommen wird.

4. Vorrichtung nach Anspruch 3, bei welcher die Einsetzposition des mindestens einen Trokars in dem Hauptbildschirmbereich durch die UI angepasst wird.

5. Vorrichtung nach Anspruch 3, bei welcher in dem Vorschauanzeigebereich ein Bild eines in den mindestens einen Trokar eingeführten Werkzeugs, das in das Innere des virtuellen Pneumoperitoneum-Modells eintritt, durch die Kamera aufgenommen und in Echtzeit angezeigt wird.

6. Vorrichtung nach Anspruch 3, bei welcher in dem Hauptbildschirmbereich die gespeicherten Entfernungsinformationen und Informationen über ein durch dem mindestens einen Trokar eingeführtes Werkzeug angezeigt werden, und
wobei in dem Vorschaubildschirmbereich Port-Nummern des Referenztrokars und des mindestens einen Trokars angezeigt werden, und angezeigt wird, ob ein entsprechendes Werkzeug mit einem inneren Organ kollidiert, wenn das entsprechende Werkzeug in das Innere des virtuellen Pneumoperitoneum-Modells an Positionen des Referenztrokars und des mindestens einen Trokars eintritt.

7. Vorrichtung nach Anspruch 2, bei welcher der Prozessor Werte von Differenzen von einem erste Punkt, welcher der Einsetzposition des Referenztrokars entspricht, zu einem zweiten Punkt, welcher der Einsetzposition des mindestens einen Trokars entspricht, in Quer-, Längs- und Diagonalrichtung berechnet, wenn die Einsetzposition des mindestens einen Trokars bestimmt wurde, eine vorgegebene Einheit von Strahlen in Quer-, Längs- und Diagonalrichtung abstrahlt, dreidimensionale Positionen, welche die Strahlen erreichen, auf der Oberfläche des virtuellen Pneumoperitoneum-Modells speichert, und die Einsetzposition des mindestens einen Trokars durch Summieren der Entfernungen der gespeicherten dreidimensionalen Positionen bestimmt.

8. Vorrichtung nach Anspruch 7, bei welcher der Prozessor beim Bestimmen der Einsetzposition des mindestens einen Trokars die Einsetzposition des mindestens einen Trokars basierend auf einer geänderten Position des zweiten Punkts ändert, wenn die Position des zweiten Punkt verändert wird.

## Revendications

1. Programme d'ordinateur combiné à un ordinateur en tant que matériel, et mémorisé dans un support d'enregistrement lisible par ordinateur pour exécuter le procédé de détermination d'une position d'insertion d'un trocart sur un modèle de pneumopéritoine virtuel tridimensionnel d'un patient, le procédé étant mis en œuvre par un dispositif, le procédé comprenant les étapes consistant à :
afficher une interface utilisateur (UI) pour définir la position d'insertion du trocart sur le modèle de pneumopéritoine virtuel utilisé lors d'une simulation de chirurgie ;
afficher le modèle de pneumopéritoine virtuel sur l'UI ;
déterminer une position espacée d'un nombril du modèle de pneumopéritoine virtuel d'une distance prédéfinie en tant que position d'insertion d'un trocart de référence, dans lequel doit être insérée une caméra (endoscope), par l'intermédiaire de l'UI ;
déterminer une position d'insertion d'au moins un trocart sur une surface du modèle de pneumopéritoine virtuel sur la base de la position d'insertion déterminée du trocart de référence, par l'intermédiaire de l'UI ;
calculer et mémoriser des informations concernant une distance entre la position d'insertion du trocart de référence et la position d'insertion déterminée de l'au moins un trocart, sur la surface du modèle de pneumopéritoine virtuel ; et
fournir les informations de distance mémorisées, sur l'UI, en temps réel pendant une chirurgie réelle sur le patient,
dans lequel les informations de distance comprennent des distances transversale et longitudinale entre la position d'insertion du trocart de référence et la position d'insertion de l'au moins un trocart, et une distance diagonale associée aux distances transversale et longitudinale.

2. Appareil pour déterminer une position d'insertion d'un trocart sur un modèle de pneumopéritoine virtuel tridimensionnel d'un patient, l'appareil comprenant :
une partie d'acquisition (110) configurée pour acquérir le modèle de pneumopéritoine virtuel utilisé lors d'une simulation de chirurgie ;
une partie d'affichage (130) configurée pour afficher une interface utilisateur (UI) pour définir la position d'insertion du trocart sur le modèle de pneumopéritoine virtuel ; et
un processeur (140) configuré pour :
afficher le modèle de pneumopéritoine virtuel sur l'UI ;
déterminer une position espacée d'un nombril du modèle de pneumopéritoine virtuel d'une distance prédéfinie en tant que position d'insertion d'un trocart de référence, dans lequel doit être insérée une caméra, par l'intermédiaire de l'UI ;
déterminer une position d'insertion d'au moins un trocart sur une surface du modèle de pneumopéritoine virtuel sur la base de la position d'insertion déterminée du trocart de référence, par l'intermédiaire de l'UI ;
calculer et mémoriser des informations concernant une distance entre la position d'insertion du trocart de référence et la position d'insertion déterminée de l'au moins un trocart, sur la surface du modèle de pneumopéritoine virtuel ; et
fournir les informations de distance mémorisées, sur l'UI, en temps réel pendant une chirurgie réelle sur le patient,
dans lequel les informations de distance comprennent des distances transversale et longitudinale entre la position d'insertion du trocart de référence et la position d'insertion de l'au moins un trocart, et une distance diagonale associée aux distance transversale et longitudinale.

3. Appareil selon la revendication 2, dans lequel l'UI comprend une zone d'écran principal (201), dans laquelle le modèle de pneumopéritoine virtuel est affiché sur une vue en plan, et une zone d'écran d'aperçu (202), dans laquelle est affichée une image interne du modèle de pneumopéritoine virtuel capturée par l'intermédiaire de la caméra insérée à travers le trocart de référence.

4. Appareil selon la revendication 3, dans lequel la position d'insertion de l'au moins un trocart est réglée sur la zone d'écran principal par l'intermédiaire de l'UI.

5. Appareil selon la revendication 3, dans lequel, dans la zone d'écran d'aperçu, une image d'un outil inséré dans l'au moins un trocart pénétrant l'intérieur du modèle de pneumopéritoine virtuel est capturée par l'intermédiaire de la caméra et est affichée en temps réel.

6. Appareil selon la revendication 3, dans lequel, dans la zone d'écran principal, les informations de distance mémorisées et des informations concernant un outil inséré à travers l'au moins un trocart sont affichées, et
dans lequel, dans la zone d'écran d'aperçu, des numéros d'orifice du trocart de référence et de l'au moins un trocart sont affichés, et le fait qu'un outil correspondant entre éventuellement en collision avec un organe interne lorsque l'outil correspondant pénètre l'intérieur du modèle de pneumopéritoine virtuel au niveau de positions du trocart de référence et de l'au moins un trocart est affiché.

7. Appareil selon la revendication 2, dans lequel le processeur calcule des valeurs de différences d'un premier point correspondant à la position d'insertion du trocart de référence à un second point correspondant à la position d'insertion de l'au moins un trocart dans des directions transversale, longitudinale et diagonale lorsque la position d'insertion de l'au moins un trocart a été déterminée, émet un rayonnement d'une unité prédéfinie de rayons dans les directions transversale, longitudinale et diagonale, mémorise des positions tridimensionnelles, qu'atteignent les rayons, sur la surface du modèle de pneumopéritoine virtuel, et détermine la position d'insertion de l'au moins un trocart en sommant les distances des positions tridimensionnelles mémorisées.

8. Appareil selon la revendication 7, dans lequel le processeur modifie la position d'insertion de l'au moins un trocart sur la base d'une position modifiée du second point lorsque la position du second point a été modifiée, lorsque que la position d'insertion de l'au moins un trocart a été déterminée.
